(19) Europäisches Patentamt
European Patent Office
Office européen des brevets

(11) EP 1 813 274 B1

(12) **EUROPEAN PATENT SPECIFICATION**

(45) Date of publication and mention of the grant of the patent:
**16.12.2009 Bulletin 2009/51**

(21) Application number: **05790185.2**

(22) Date of filing: **05.10.2005**

(51) Int Cl.:
***A61K 31/4422*** *(2006.01)* ***A61K 47/02*** *(2006.01)*
***A61K 47/36*** *(2006.01)* ***A61K 9/16*** *(2006.01)*
***A61K 9/20*** *(2006.01)*

(86) International application number:
**PCT/JP2005/018474**

(87) International publication number:
**WO 2006/038661 (13.04.2006 Gazette 2006/15)**

(54) **MEDICINAL COMPOSITION, PROCESS FOR PRODUCING THE SAME, AND METHOD OF STABILIZING DIHYDROPYRIDINE COMPOUND IN MEDICINAL COMPOSITION**

MEDIZINISCHE ZUSAMMENSETZUNG, VERFAHREN ZU IHRER HERSTELLUNG UND VERFAHREN ZUR STABILISIERUNG EINER DIHYDROPYRIDIN-VERBINDUNG IN EINER MEDIZINISCHEN ZUSAMMENSETZUNG

COMPOSITION MEDICINALE, PROCEDE DE PRODUCTION DE CELLE-CI, ET METHODE DE STABILISATION D'UN COMPOSE DE DIHYDROPYRIDINE DANS UNE COMPOSITION MEDICINALE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.10.2004 JP 2004293771**

(43) Date of publication of application:
**01.08.2007 Bulletin 2007/31**

(73) Proprietor: **Eisai R&D Management Co., Ltd.**
**Tokyo 112-8088 (JP)**

(72) Inventors:
- **SUZUKI, Toshio,**
  **EISAI CO., LTD.**
  **Honjo-shi, Saitama 367-0048 (JP)**
- **YASUI, Masanobu,**
  **EISAI CO., LTD.**
  **Honjo-shi, Saitama 367-0048 (JP)**
- **SUGAWARA, Satoshi,**
  **EISAI CO., LTD.**
  **Honjo-shi, Saitama 367-0048 (JP)**
- **MORITA, Yutaka,**
  **EISAI CO., LTD.**
  **Honjo-shi, Saitama 367-0048 (JP)**

(74) Representative: **HOFFMANN EITLE**
**Patent- und Rechtsanwälte**
**Arabellastrasse 4**
**81925 München (DE)**

(56) References cited:
**WO-A1-01/64190** **WO-A1-03/059330**
**JP-A- 57 058 609** **JP-A- 58 083 617**
**JP-A- 2000 103 731** **JP-A- 2000 191 516**
**JP-A- 2002 539 238** **JP-A- 2003 104 888**
**JP-B2- 3 179 658**

- **DESAI D.S. ET AL: 'Photostabilization of uncoated tablets of sorivudine and nifedipine by incorporation of synthetic iron oxides' INTERNATIONAL JOURNAL OF PHARMACEUTICS vol. 103, no. 1, 25 February 1994, pages 69 - 76, XP001085020**
- **DESAI D.S. ET AL: 'Photo-stabilization of the tablets of antiviral drug SQ32756 (BV-araU) and nifedipine by incorporation of synthetic iron oxides' PHARMACEUTICAL RESEARCH vol. 8, no. 10 (SUPPL), October 1991, page S-176, XP001080067**
- **MATSUMOTO M. ET AL: 'YAKUGAKU MANNUAL (NANZANDO) 1st edition', 20 March 1989 pages 88 - 92, XP002998991**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 813 274 B1

## Description

Technical Field

The present invention relates to pharmaceutical compositions comprising amlodipine besylate and at least one selected from yellow ferric oxide, red ferric oxide and carrageenan; a method for producing the pharmaceutical compositions; and a method for stabilizing amlodipine besylate in the pharmaceutical compositions.

Background Art

Most of advanced countries like Japan are rapidly aging; allegedly, no less than half of hypertension patients are old persons of no less than seventy years old. It is undesirable for old persons to forcibly suppress blood pressure, therefore, hypotensive agents should be administered in a cautious manner if employed. As such, dihydropyridine calcium antagonists are typically selected for the first drug of old hypertension patients in view of its mild effect and continuousness. For example, film-coated tablets containing amlodipine besylate, granules containing aranidipine, fine granules of nifedipine, and uncoated tablets of manidipine hydrochloride are conventional candidates of pharmaceutical compositions containing a dihydropyridine compound associated with calcium antagonism. These formulations are typically of film-coated tablets or granules or of colored-substrate capsules, alternatively, necessity to reserve under light-shielding condition is denoted since such dihydropyridine compounds are typically photodegradable (see Patent Literature 1). In some cases, dihydropyridine compounds decrease their content and/or increase impurities upon contact with additives within pharmaceutical compositions. For example, it is also reported that lactose, one of conventional excipients, should be avoided to formulate with since it decomposes amlodipine besylate (see e.g., Patent Literature 2, Non-Patent Literature 1). For the countermeasure, for example, a tablet is disclosed that comprises a crystalline cellulose formulated into a pharmaceutical composition at a higher content of 87% to 94% (see e.g., Patent Literature 2).

On the other hand, tablets or capsules of pharmaceutical products are mostly produced in an individual amount per dose, thus a number of automatic dose unit packaging machines have been introduced into preparation for pharmaceutical products. However, tablets are inevitably reserved for a while into non-packaged tablet cases on the automatic tablet packaging machines; as such, it is reported that storage conditions such as lights, humidities, and higher temperatures may degrade or discolor them (see e.g., Non-Patent Literature 2). Specifically, rapidly disintegrating tablets with easier dosing acceptance and/or granules with easier dosing adjustability are difficult to be coated with colorants, thus are likely to be influenced by storage conditions such as lights and moisture. Accordingly, most of them typically alarm to limit their handling manners into those under light-shielding etc. by way of denoting on package containers, for example, instructing additionally to reserve under light-shielding with respect to granules even coated with a coating agent.

Since the dihydropyridine compounds are unstable due to promotional oxidation under lights or heat and also decrease their assay depending on co-existing excipients, as described above, there are no more than limited additives, dosage forms, or packaging forms possible for pharmaceutical compositions containing dihydropyridine compounds. In addition, widely-used automatic tablet packaging machines require stable pharmaceutical compositions containing dihydropyridine compounds to be free from influence of dispensing and/or storage conditions even under unpackaged conditions. Moreover, the pharmaceutical compositions containing dihydropyridine compounds are often administered to old hypertension patients, therefore, it is desirable to provide a dosage form that allows to easily administer to patients with poor swallowing ability and/or to easily adjust the dosage so as to bring about careful administration for old persons.

Patent Literature 1: Japanese Patent Application Laid-Open (JP-A) No. 2003-104888
Patent Literature 2: International Publication No. WO 03/051364
Non-Patent Literature 1: M. Abdoh et al, Amlodipine Besylate-Excipients Interaction in Solid Dosage Form, Pharmaceutical Development and Technology, USA, 2004, vol. 9, No. 1, pp.15-24;
Non-Patent Literature 2: Hiroshi Yuasa, et al., Tablet Discoloration under Light as well as BHT, Hospital and Pharmacy, 1995, vol. 21, No. 3, pp. 231-242

The present invention aims to solve the problems as regards pharmaceutical compositions in the art and to attain the objects shown below. That is, it is an object of the present invention to improve the stability of dihydropyridine compounds in pharmaceutical compositions under influence of additives and storage conditions and to provide stable pharmaceutical compositions containing dihydropyridine compounds with superior handling ability and affordable administration.

More specifically, it is an object of the present invention to provide pharmaceutical compositions containing stable dihydropyridine compounds that are not significantly limited in terms of additives, dosage forms, or packaging and are substantially free from influence of formulation processing and/or storage conditions even under unpackaged conditions.

It is another object of the present invention to provide pharmaceutical compositions containing dihydropyridine compounds with a dosage form that allows to easily administer to patients with poor swallowing ability and/or to easily adjust the dosage so as to bring about careful administration for old persons.

Desai D.S. et al: "Photostabilization of uncoated tablets of sorivudine and nifedipine by incorporation of synthetic iron oxides "INTERNATIONAL JOURNAL OF PHARMACEUTICS vol. 103, no. 1, 25 February 1994, pages 69-76, XP001085020 and Desai D.S. et al: "Photostabilization of the tablets of antiviral drug SQ32756 (BV-araU) and nifedipine by incorporation of synthetic iron oxides" PHARMACEUTICAL RESEARCH vol. 8, no. 10 (Suppl).; October 1991, page S-176, XP001080067 disclose a tablet comprising 0.2% (w/w) yellow iron oxide and nifedipine, respectively.

JP 57 058 609 A JP 2000 191516 A discloses a pharmaceutical composition comprising nifedipine and carrageenan.

JP 2003 104888 A discloses a tablet comprising iron oxide and a dihydropyridine compound.

Disclosure of Invention

The inventors have investigated vigorously to solve the problems described above and have found that inclusion of at least one selected from yellow ferric .oxide, red ferric oxide and carrageenan into pharmaceutical compositions can remarkably stabilize amlodipine besylate, more specifically, at least one selected from yellow ferric oxide, red ferric oxide and carrageenan can suppress impurities derived from oxidation of amlodipine besylate Furthermore, the inventors have found that the pharmaceutical compositions containing amlodipine besylate can maintain a pharmaceutical quality and provide easier dosing acceptance and/or easier dosing adjustability even without applying an especial dampproof and/or light-shielding coating on drug products nor employing a dampproof and/or light-shielding package for drug products, consequently the present invention has been completed.

Accordingly, the present invention provides pharmaceutical compositions that can secure the stability of dihydropyridine compounds to storage conditions such as temperatures by including at least one selected from iron oxide and carrageenan. Moreover, the present invention provides methods for stabilizing dihydropyridine compounds within pharmaceutical compositions. Furthermore, the present invention provides a novel use of at least one selected from iron oxide and carrageenan as a stabilizing agent of the dihydropyridine compounds.

The present invention has been made based on the above-noted findings of the present inventors; the problems can be solved by the invention described below. That is, the present invention is:

<1> A pharmaceutical composition, comprising a dihydropyridine compound, and at least one selected from iron oxide and carrageenan.

<2> The pharmaceutical composition described in <1>, wherein the dihydropyridine compound and at least one selected from the iron oxide and carrageenan are mixed.

<3> The pharmaceutical composition described in <1> or <2>, wherein the dihydropyridine compound is amlodipine besylate.

<4> The pharmaceutical composition described in any one of <1> to <3>, wherein the iron oxide comprises at least one selected from yellow ferric oxide and red ferric oxide.

<5> The pharmaceutical composition described in any one of <1> to <4>, wherein the content of the iron oxide is 0.05 part by mass to 8 parts by mass based on 1 part by mass of the dihydropyridine compound.

<6> The pharmaceutical composition described in any one of <1> to <5>, wherein the iron oxide is yellow ferric oxide.

<7> The pharmaceutical composition described in <6>, wherein the content of the yellow ferric oxide is 0.05 part by mass to 1 part by mass based on 1 part by mass of the dihydropyridine compound.

<8> The pharmaceutical composition described in any one of <1> to <7>, wherein the content of the carrageenan is 0.05 part by mass to 1.2 parts by mass based on 1 part by mass of the dihydropyridine compound.

<9> The pharmaceutical composition described in any one of <1> to <8>, wherein the pharmaceutical composition is one of fine granules, granules, tablets, capsules, and dry syrup.

<10> The pharmaceutical composition described in <9>, wherein the pharmaceutical composition is tablets produced by a method comprising:

(i) mixing a dihydropyridine compound and at least one selected from iron oxide and carrageenan to prepare a mixture, and

(ii) dry-compressing the mixture into tablets.

<11> The pharmaceutical composition described in <9> or <10>, wherein the tablets are rapidly disintegrating tablets.

<12> A method for producing a pharmaceutical composition, comprising:

(i) mixing a dihydropyridine compound and at least one selected from iron oxide and carrageenan to prepare a mixture, and
(ii) dry-compressing the mixture into tablets.

In accordance with the present invention, a pharmaceutical composition containing a amlodipine besylate can be provided, in which the stability of the amlodipine besylate is improved in the pharmaceutical composition under influence of additives and storage conditions, and also the pharmaceutical composition represents superior handling ability and affordable administration.

More specifically, the present invention provides the pharmaceutical composition containing amlodipine besylate in which the amlodipine besylate can maintain its stability to lights, moistures, or temperatures even in such dosage forms as having higher surface areas like granules and fine granules or as rapidly disintegrating tablets to which coating being typically unfeasible, even without employing an especial coating agent, a capsule material, or a package. Therefore, the present invention provides a pharmaceutical composition that can maintain higher quality with substantially no possibility to decrease the content of the amlodipine besylate induced by such reasons as storage conditions of pharmaceutical products till administering to patients, operating conditions to dispense using automatic dose unit packaging machines, and breakage or comminution of granules or tablets while their delivery or preparation. In addition, the present invention provides a pharmaceutical composition that can lead to oral rapidly-disintegrating tablets allowing to easily administer to patients with poor swallowing ability or that can lead to granules or scored tablets allowing to easily adjust the dosage levels finely depending on symptom or age of patients.

In addition, the present invention provides a method for producing a pharmaceutical composition, in which there needs no light-shielding coating on the pharmaceutical composition in order to stabilize the amlodipine besylate the production steps are simplified, and thus the productivity can be enhanced.

Moreover, the present invention provides a method for stabilizing amlodipine besylate.

Brief Description of Drawings

FIG. 1 is a graph that shows a change of impurity content with time in granules containing amlodipine besylate under 40°C and relative humidity 75% (open condition).
FIG. 2 is a graph that shows a change of impurity content with time in granules containing amlodipine besylate under 60°C (closed condition).
FIG. 3 is a graph that shows a change of impurity content relative to compounding ratios of iron oxide to amlodipine besylate when a storage stability test was carried out using a rapidly disintegrating tablet containing amlodipine besylate.
FIG. 4 is a graph that shows a change of impurity content relative to compounding ratios of carrageenan to amlodipine besylate when a storage stability test was carried out using a rapidly disintegrating tablet containing amlodipine besylate.
FIG. 5 is a graph that shows a change of impurity content relative to compounding ratios of yellow ferric oxide to amlodipine besylate when a storage stability test was carried out using a tablet containing amlodipine besylate produced by direct dry-compressing.
FIG. 6 is a graph that shows a change of impurity content relative to compounding ratios of red ferric oxide to amlodipine besylate when a storage stability test was carried out using a tablet containing amlodipine besylate produced by direct dry-compressing.
FIG. 7 is a graph that shows a change of impurity content relative to compounding ratios of yellow ferric oxide to benedipine hydrochloride when a storage stability test was carried out using a tablet containing benedipine hydrochloride produced by direct dry-compressing.

Best Mode for Carrying Out the Invention

The present invention will be explained with reference to its embodiments. The embodiments shown below are exemplified for the purpose of no more than explanation of the present invention, thus to which the present invention will be limited in no way; namely, the present invention can be implemented in various embodiments without departing from its gist. Pharmaceutical Composition

The pharmaceutical composition according to the present invention comprises amlodipine besylate and at least one selected from yellow ferric red ferric oxide and carrageenan, and also other optional ingredients as required.

The content of the dihydropyridine compound in the pharmaceutical composition may be properly selected as long as able to administer an effective amount to patients; preferably, the content is 0.01 to 20 parts by mass based on 100 parts by mass of the pharmaceutical composition, more preferably 0.05 to 15 parts by mass, still more preferably 0.1 to 10 parts by mass.

Iron Oxide

The iron oxide is red ferric oxide ($Fe_2O_3$), yellow ferric oxide ($Fe_2O_3$-$H_2O$), or mixtures thereof.

The red ferric oxide and yellow ferric oxide may be of naturally occurring pigments that are listed in Pharmaceutical Additives Standard 2003 (hereinafter referred to as "PAS") and have been conventionally used for colorant. The red ferric oxide is a powder of red, auburn or dark red-purple color, and the yellow ferric oxide is a powder of yellow or brownish yellowish ocher color. Both of them are very water-insoluble.

The red ferric oxide may be properly selected depending on the application, an example thereof is Red ferric oxide (article name, by Kishi Chemical Co.). The yellow ferric oxide may also be properly selected depending on the application, examples thereof are Yellow Ferric Oxide Colorcon (article name, by Japan Colorcon Co.) and Yellow Ferric Oxide (article name, by Junsei Chemical Co.).

In the present invention, these iron oxides may be used alone or in combination thereof.

The content of the iron oxide in the pharmaceutical composition, properly selected depending on the application, is preferably 0.05 to 8 parts by mass based on 1 part by mass of the dihydropyridine compound, more preferably 0.08 to 5 parts by mass, still more preferably 0.1 to 5 parts by mass.

In cases where the yellow ferric oxide is solely used as the iron oxide, the content of the yellow ferric oxide is preferably 0.05 to 1 part by mass based on 1 part by mass of the dihydropyridine compound in the pharmaceutical composition, more preferably 0.08 to 1 part by mass, still more preferably 0.1 to 0.5 part by mass from the viewpoint that the yellow ferric oxide affords relatively higher stabilizing effect among the iron oxides described above. Carrageenan

The carrageenan, properly selected from various carrageenans depending on the application, is preferably i-earrageenan, κ-carrageenan, or λ-carrageenan in particular.

Such carrageenans are a type of polysaccharides utilized widely in pharmaceutical and food fields. It is publicly known that there exist various types of carrageenans such as i-carrageenan, κ-carrageenan, and λ-carrageenan, and also biological precursors thereof such as μ-carrageenan and v-carrageenan.

The carrageenan may be those commercially available from, for example, FMA Corporation (USA), Sansho Co., Ltd., or the like.

The content of the carrageenan in the pharmaceutical composition may be properly selected depending on the application; preferably, the content is 0.05 to 1.2 parts by mass based on 1 part by mass of the dihydropyridine compound in the pharmaceutical composition, more preferably 0.05 to 1.0 part by mass, still preferably 0.1 to 0.8 part by mass, and still more preferably 0.2 to 0.5 parts by mass.

The inventive pharmaceutical composition of dihydropyridine compound may contain one of iron oxide and carrageenan or both of iron oxide and carrageenan. In cases where both of iron oxide and carrageenan are contained, the total content of them may be appropriately selected without particular limitation; preferably, the respective contents of iron oxide and the carrageenan are within the range described above based on the dihydropyridine compound in the pharmaceutical composition.

Other Ingredients or additives

The pharmaceutical composition may contain, in addition to the ingredients described above, such additives as excipients, binders, lubricants, disintegrating agents, coating agents, plasticizers, suspending agents, emulsifying agents, flavoring agents, oxidation inhibitors, sugar coating, moisture-proof agents, fluidizing agents and colorants in a range where the effects of the present invention being not despaired. The additives may be optionally selected other than these additives.

The excipients may be properly selected depending on the application; examples thereof include D-mannitol, white soft sugar, sucrose, sodium bicarbonate, corn starch, potato starch, wheat starch, rice starch, partly pregelatinized starch, crystalline cellulose, light anhydrous silicic acid, anhydrous calcium phosphate, precipitated calcium carbonate, and calcium silicate.

The binder may be properly selected depending on the application; examples thereof include povidone, dextrin, hydroxypropylcellulose, hydroxypropylmethylcellulose, methylcellulose, polyvinylalcohol, sodium carboxymethylcellulose, alphanized starch, sodium alginate, pullulan, and acacia powdered.

The lubricant may be properly selected depending on the application; examples thereof include hydrogenated oil, hydrogenated castor oil, stearic acid, magnesium stearate, calcium stearate, glyceride behenate, and sodium stearyl fumarate.

The disintegrating agent may be properly selected depending on the application; examples thereof include low-substituted hydroxypropylcellulose, carmellose, sodium carboxymethyl starch, and crospovidone.

The coating agent may be properly selected depending on the application; examples thereof include cellulose derivatives such as hydroxypropyl cellulose, hydroxypropylmethyl cellulose, ethylcellulose, hydroxypropylmethyl cellulose phthalate, carboxymethylethyl cellulose, sodium carmellose, potassium carmellose, cellulose acetate, and cellulose acetate phthalate; acrylic acid copolymers such as ethylacrylate methylmethacylate copolymers dispersions, aminoalkylmethacrylate copolymer E, aminoalkylmethacrylate copolymer RS, methacrylic acid copolymer L, methacrylic acid copolymer LD, methacrylic acid copolymer S, 2-methyl-5-vinylpyridinemethylacrylate methacrylic acid copolymer, dimethylamino ethylmethacrylate methylmethacrylate copolymer; synthetic polymers such as polyvinylpyrrolidone, polyvinylacetal diethylamino acetate, polyvinylalcohol, polyoxyethylene polyoxypropylene glycol, and macrogol; polysaccharides such as pullulan and chitosan; natural-occurring polymers such as gelatin, gelatin succinate, gum acacia, and shellac.

The plasticizer may be properly selected depending on the application; examples thereof include dioctyl adipate, triethyl citrate, triacetin, glycerin, concentrated glycerin, and propyleneglycol.

The suspending or emulsifying agent may be properly selected depending on the application; examples thereof include lecithin, sucrose fatty ester, polyglycerin fatty ester, polyoxyethylene hydrogenated castor oil, polysorbate, and polyoxyethylene polyoxypropylene copolymer.

The flavoring agent may be properly selected depending on the application; examples thereof include menthol, peppermint, lemon oil, and orange oil.

The antioxidant may be properly selected depending on the application; examples thereof include sodium ascorbate, L-cysteine, sodium sulfite, nature vitamin E, dibutylhydroxytoluene, and hydroxyanisole.

The sugar coating may be properly selected depending on the application; examples thereof include white soft sugar, lactose, starch syrup, precipitated calcium carbonate, gum acacia, carnauba wax, shellac, beeswax, macrogol, ethylcellulose, methylcellulose, and povidone.

The moisture-proof agent may be properly selected depending on the application; examples thereof include magnesium silicate, light anhydrous silicic acid, hydrogenated oil, stearic acid, magnesium stearate, paraffin, castor oil, macrogol, vinyl acetate resin, cellulose acetate phthalate, polyvinylacetal diethylamino acetate, and shellac.

The glidants may be properly selected depending on the application; examples thereof include hydrous silicon dioxide, light anhydrous silicic acid, heavy anhydrous silicic acid, crystalline cellulose, synthetic aluminum silicate, aluminum magnesium hydroxide, magnesium aluminometasilicate, stearic acid, calcium stearate, magnesium stearate, tricalcium phosphate, talc, and corn starch.

The colorant may be properly selected depending on the application; examples thereof include Tar colorants such as Food Yellow No. 4, Food Yellow No. 5, Food Red No. 2, Food Red No. 102, Food Blue No. 1, Food Blue No. 2 (indigo carmine), and Food Yellow No. 4 aluminum Lake; titanium oxide, zinc oxide, talc, Curcuma extract, caramel, carotene liquid, beta-carotene, copper chlorophyll, sodium copper chlorophyllin, riboflavin, carbon black, and medical carbon.

The additives described above as the other ingredients may be used alone or in combination. The content of the other ingredient may be properly selected in the pharmaceutical composition in a range where the effects of the present invention being not despaired.

Dosage Form

The dosage form of the inventive pharmaceutical composition may be properly selected depending on the application; for example, the dosage form may be of oral solid formulations; specific examples thereof include powdered drug, fine granules, granules, capsules, pills, tablets, troche, dry syrup, and the like. Among these, preferable are powdered drug, fine granules, granules, tablets, capsules, and dry syrup, more preferable are fine granules, granules, and tablets. It is preferred in particular that the tablets are those of rapidly disintegrating. The rapidly disintegrating tablets in the present invention refer to those disintegrating remarkably promptly, typically disintegrating within one minute in oral cavities where saliva moisture exists in a significantly small amount. Such rapidly disintegrating tablets are suitable for children and/or old people having less capacity to take tablets.

Production Method

The production method of the inventive pharmaceutical composition may be properly selected depending on the application, illustrative methods for the preferable dosage forms are shown below.

As described above, the inventive pharmaceutical composition may contain one or both of iron oxide and carrageenan. The production method of the inventive pharmaceutical composition, containing both of iron oxide and carrageenan, may be properly selected in terms of mixing manner and additive order without limitation, that is, the iron oxide and the carrageenan may be added separately in any order or the both may be previously mixed and then added together.

Powdered Drug

The pharmaceutical composition may also be produced into a powdered drug through mixing a dihydropyridine compound at least one selected from the iron oxide and carrageenan, and adding optional other ingredients such as excipients.

Fine Granules and Granules

The pharmaceutical composition may also be produced into powder drug or granules through mixing a dihydropyridine compound, at least one selected from the iron oxide and carrageenan, and optional other ingredients such as excipients and binders to pelletize them into a granulated material. In addition, the granulated material may be optionally added with additives such as flavoring agents to produce fine granules or granules.

Capsules

The pharmaceutical composition may also be produced into capsules through, for example, filling the granulated material described above into capsules based on gelatin or hydroxypropylmethylcellulose.

Dry Syrups

The pharmaceutical composition may also be produced into dry syrups through, for example, adding to the granulated material described above additives such as suspending agents, saccharides, and flavoring agents. Tablets

The pharmaceutical composition may be also produced into tablets through mixing granules, containing a dihydropyridine compound and at least one selected from the iron oxide and carrageenan, with optional other ingredients such as lubricant and disintegrating agents and then tabletting them.

The fine granules, granules, or tablets described above may be applied with a coating such as of enteric film and sugar film as required.

Exemplary Production Method (i) for Fine Granules or Granules

The method for producing fine granules or granules of the inventive pharmaceutical composition will be explained specifically in the following.

The fine granules or granules may be produced by way of a conventional method or combinations of conventional methods. For example, a granulating process is essential for production methods for fine granules or granules, and also the other processes such as of mixing, drying, sizing and classifying may be combined as required.

The granulating process may be wet-granulating processes in which a powder is added with a binder and a solvent then granulated, dry-granulating processes in which a powder is press-granulated, or melt-granulating processes in which a powder is mixed with a heat-melting binder and then heat-granulated. These granulating processes may be combined with various granulating processes such as agitating granulation method used with machines such as planetary mixers and screw mixers, high shear granulation method used with machines such as Henschel mixers and super mixers, extrusion granulation method used with machines such as cylindrical,rotary granulator, screw-extruding granulator and pellet-mill granulator, or other processes like, tumbling-granulation method, fluidized-bed granulation method, compression granulation method, crushing granulation method, and spray dry granulation method

Following producing granules by way of the granulating processes, the intermediate products are subjected to drying, crushing or loosing, and size-controlling thereby the inventive fine granules or granules may be produced.

More specifically, the inventive fine granules or granules may be produced, for example, by way of mixing a dihydropyridine compound, an excipient, and a binder by use of a mixer or a kneader, adding iron oxide or carrageenan or the mixture thereof, mixing or kneading the mixture, then pelletizing granulates by use of an extrusion granulator. The extrusion granulator, properly selected depending on the application, may be a basket-type granulator or cylindrical granulator. The resulting pellets are dried by use of a shelf-type drier or a fluidized-bed drier and size-controlled by use of a mill or an oscillator thereby to produce a fine granules or granules.

Alternatively, the inventive fine granules or granules may be produced, for example, by way of pressing and molding a dihydropyridine compound, an excipient, and a lubricant, and also iron oxide or carrageenan or mixture thereof, while stirring and mixing them, by use of dry-press compressors such as roller compactors and slug-tableting machines then crushing into an appropriate size. The resulting pellets, prepared by these pelletizers, may be formed into the inventive fine granules or granules without additional processes, or crushed and size-controlled by use of power mills, roll granulators, or rotor speed mills thereby to produce the inventive fine granules drug or granules.

In the granulation process described above, a solvent for granulating may be utilized additionally. The solvent for granulating, selected properly depending on the application, may be water or organic solvents, more specifically, water, lower alcohols such as methanol and ethanol, ketones such as acetone, methylethylketone, methylene chloride or mixtures thereof, for example.

Exemplary Production Method (ii) for Tablets

The method for producing tablets of the inventive pharmaceutical composition will be explained specifically in the following.

The tablets may be produced by way of a conventional method or combinations of conventional methods. For example, a tabletting process is essential for production methods of tablets, and also the other processes such as of mixing, drying, and coating like sugar-coating may be combined as required. The tabletting process may be, for example, a direct compression method where dihydropyridine compounds and pharmaceutically acceptable additives are mixed and then the mixture is compressed into tablets by use of tabletting machines, or a wet granule-compression method or dry granule-compression method where granules are prepared by a similar process as that of the fine granules drug or granules to which a lubricant or a disintegrating agent is mixed as required, then the mixture is compressed into tablets. The tabletting machine for compression to produce tablets may be, for example, single-punch tabletting machines, rotary tabletting machines, or core-containing tabletting machines.

The inventive tablets may be appropriately produced by use of excipients such as mannitol, binders such as polyvinyl pyrrolidone and crystalline cellulose, disintegrating agents such as sodium carmellose and crospovidone, and lubricants such as magnesium stearate and talc, and the mixture is compressed into tables as described above.

Exemplary Production Method (iii) for Rapidly Disintegrating Tablets

The pharmaceutical composition of the inventive pharmaceutical composition may be produced into rapidly disintegrating tablets for improving administering property. The rapidly disintegrating tablets are a preferable embodiment of the inventive tablets as described above. The illustrative production processes of the inventive pharmaceutical composition for the rapidly disintegrating tablets will be explained in the following.

The inventive rapidly disintegrating tablets may be produced, for example, by way of adding mannitol, polyvinyl pyrrolidone and at least one selected from iron oxide and carrageenan to a dihydropyridine compound to prepare a mixture, to which then water and a water-soluble solvent are mixed and the resulting mixture is kneaded. Then the kneaded mixture is molded into tablets by use of a machine disclosed, for example, in Japanese Patent (JP-B) No. 3179658 and then drying them. In the machine disclosed in JP-B No. 3179658, tablets are punched with a film at molding, thus wet powders can be formed into tablets without adhering to punch; as such the machine is one of most suited machines to produce the inventive rapidly disintegrating tablets. The machine may typically provide tablets with a disintegrating period of no longer than 30 seconds within oral cavities.

Application

The inventive pharmaceutical composition may be adequately applied to treat or to remedy hypertension, renoparenchymal hypertension, renovascular hypertension, angina pectoris, variant angina pectoris and the like.

Dosage

When treatment is intended for the diseases described above, the dosage of the dihydropyridine compound administered with the pharmaceutical composition may be appropriately adjusted depending on ages or conditions of the patients. The dosage for adult humans is typically 0.1 to 250 mg per day, preferably 1 to 125 mg per day as the amount of the dihydropyridine compound or its pharmaceutically acceptable salts. The dosage per day may be administered by one or plural times. For example, the amount per dose of the dihydropyridine compound or its pharmaceutically acceptable salts is typically 0.01 to 250 mg, preferably 0.1 to 125 mg in the pharmaceutical composition.

Stabilizing Method/Stabilizing Agent

The method for stabilizing a dihydropyridine compound in the inventive pharmaceutical composition may be achieved by way of mixing the dihydropyridine compound and at least one selected from the iron oxide and carrageenan. As such, it is unnecessary to limit the mixed condition definitely of the dihydropyridine compound and the iron oxide and/or carrageenan as long as co-existing condition being attained.

The iron oxide or the carrageenan may be solely included into the pharmaceutical composition or the both may be included into the pharmaceutical composition. In cases where both of the iron oxide and the carrageenan are utilized in the present invention, the iron oxide and the carrageenan may be individually utilized apart from their use process or order, or the both may be pre-mixed and used.

In addition, at least one of the iron oxide and the carrageenan can suppress the increase of impurities derived from the dihydropyridine compound in the pharmaceutical composition, thus are effective for a stabilizing agent.

## Examples

The present invention will be explained with reference to Production Examples and inventive Examples, to which the present invention should be limited in no way. The additives in the pharmaceutical composition are those adapted to official compendia such as of Japanese Pharmacopoeia, Pharmaceutical Additives Standards, Japanese Standards for Pharmaceutical Ingredients 1997, and Food Additives, or reagents.

### Production Example 1 (Reference)

169.2 g of mannitol and 112.8 g of crystalline cellulose are mixed well to make a mixture, to which 64 g of azelnidipine and 4 g of yellow ferric oxide are added and mixed well. The mixture is formed into tablets by use of a rotary tabletting machine while externally lubricating by magnesium stearate in an amount of 0.05 part by mass per 99.5 parts by mass of the mixture, thereby to prepare rapidly disintegrating tablets each containing 16 mg of azelnidipine and having a unit mass of 180 mg.

### Production Example 2 (Reference)

25 g of felodipine, 8655 g of lactose, 1000 g of D-mannitol, 300 g of polyvinylpyrrolidone, 7.5 g of carrageenan, and 2.5 g of yellow ferric oxide are mixed well to prepare a mixture, to which 600 g of ethanol is gradually added while stirring the mixture by use of a kneader. The kneaded mixture is molded into columnar pellets by use of a cylindrical granulator with 0.5 mm screen, then the columnar pellets are dried within a fluidized-bed drier at 60°C and screened through a mesh thereby to prepare granules. Addition of 1 g of calcium stearate per 99 g of the granules may bring about granules that contain 2.5 mg of felodipine per 1000 mg of its formulation.

Test methods in Examples are shown below that were employed to examine the availability of the inventive pharmaceutical composition. Measurement of Impurity Content (i)

Impurity contents of the pharmaceutical compositions containing amlodipine besylate in Test Examples 1 to 7 were measured in accordance with a concentration inclination control or a gradient method in liquid chromatography referred by Japanese Pharmacopoeia. Assay for impurities was calculated based on an area percentage method or a relative area method. Liquid chromatography (HPLC) was measured by use of D-7000 (by Hitachi Co.) under the following conditions.

### HPLC Conditions:

detector: UV absorption spectrophotometer (wavelength: 241 nm), column: Inertsil ODS-2 4.6 mm×15 cm, particle size: 5 μm, column temperature: 40 °C, flow rate: 1.0 mL/min, injection amount: 10 μL, analysis period: 45 min, HPLC mobile phase A: acetonitrile/water/$HClO_4$ (100:900:1), HPLC mobile phase B: acetonitrile/water/$HClO_4$ (900:100:1), gradient program: shown in Table 1.

### Preparation of Sample Solution

A weighed sample was dissolved into a solution of 30% acetonitrile in a concentration of 0.7 mg/mL as the drug substance. The resulting solution was centrifuged (3000 rpm, 10 min) and/or filtered (except for initial flow of 4 mL) and to use as a sample solution.

Table 1

| Time (min) | 0 | 15 | 35 | 35.01 | 45 |
|---|---|---|---|---|---|
| Concentration of Mobile Phase B (%) | 30 | 30 | 100 | 30 | stop |

### Stability Test

A transparent glass bottle (closed or open) or a dish was used for a storage container. A test sample was uniformly spread on the dish. The constant temperature humidity baths utilized in the preservation stability test were as follows:

5°C: Medical Cooling Unit (article name, by Sanyo Co.),
40°C and 75% relative humidity (hereinafter referred to as "RH"): HIFLEX (article name, by ETAC Co.), FX230p (by ETAC Co.),
60°C: DN94 (article name, by Yamato Co.), and
light irradiation: CTX01 (article name, by Nagano Science Co.), total illuminance 1,200,000 lux.h, total near-UV radiation energy 200 W.h/$m^2$ (by ICH guide line and Pharmaceutical Council No. 422).

Examples 1 to 2

5 g of amlodipine besylate (by Dr.Reddy's Co.) and 5 g of yellow ferric oxide (by Junsei Chemical Co.) were uniformly mixed using a pestle in a mortar, to which an ethanol solution was added and the mixture was kneaded. The kneaded mixture was dried within a shelf-type drier (model DAE-20, hot-air drier, by Sanwa Kaki Industries, Co.) at 40°C for 10 hours to prepare granules of Example 1.

Granules of Example 2 were prepared in the same manner as Example 1, except that 5 g of amlodipine besylate and 5 g of carrageenan (by Sansho Co.) were used.

Reference Example 1

Granules containing solely amlodipine besylate were prepared in the same manner as Example 1 except that yellow ferric oxide was not used.

Test Example 1

The granules of Example 1, the granules of Example 2 and the granules of Reference Example 1 were stored for one month under 5°C with closed conditions, 40°C and 75% RH with open conditions, or 60°C with closed conditions respectively, then the impurity contents were measured. The results are shown in Table 2. From the results, it was confirmed that the granules of Example 1, formulated with yellow ferric oxide, showed impurity contents of below detectable limit under all conditions of temperatures and humidities and thus almost no amlodipine besylate decomposed. It is also confirmed that the granules of Example 2, formulated with carrageenan, was more stable than the granules of Reference Example 1.

In addition, it was confirmed that the granules of Example 1 and the granules of Example 2, formulated with iron oxide or carrageenan, are more stable than the granules of Reference Example 1 under the condition of light irradiation. The results of these tests demonstrate that formulation of yellow ferric oxide or carrageenan with a dihydropyridine compound may provide a stable pharmaceutical composition substantially free from influence of storage conditions such as temperature, humidity and light.

Table 2

| | Additive | 5°C closed | 40°C 75%RH Open | 60°C closed | Light Irradiation |
|---|---|---|---|---|---|
| Ex. 1 | Yellow Ferric Oxide | * | * | * | 0.05% |
| Ex.2 | Carrageenan | * | * | 0.10% | 0.66% |
| Ref. Ex.1 | non-compounded | 0.10% | 0.05% | 0.21% | 2.19% |
| | * below detectable limit | | | | |

Examples 3 to 5

0.1 g of amlodipine besylate, 4.85 g of mannitol (Towa Chemical Industry Co.), and 0.03 g of yellow ferric oxide were mixed in a mortar, to which then an ethanol solution with polyvinylpyrrolidone (hereinafter referred to as "PVP-K30", by ISP Co.) was added and the mixture was kneaded. The kneaded mixture was dried within a shelf-type drier at 50°C for 3 hours thereby to prepare granules of Example 3. In the same manner as Example 3, granules of Example 4 were prepared except that 0.1 g of amlodipine besylate, 4.85 g of mannitol, and 0.03 g of red ferric oxide (by Kishi Chemical Co.) were used. Furthermore, in the same manner as Example 3, granules of Example 5 were prepared except that 0.1 g of amlodipine besylate, 4.84 g of mannitol, and 0.20 g of carrageenan (by Sansho Co.) were used.

Comparative Example 1 to 4, Reference Example 2

Granules containing amlodipine besylate were prepared in the same manner as Example 3 except that the additives shown in Table 3 were used as stabilizing agents. The additives were ascorbic acid (referred to as "Asc", by Daiichi Pharmaceutical Co.) conventionally utilized as a stabilizing agent in solid compositions, magnesium hydroxide (referred to as "Mg hydroxide", by Kyowa Chemical Industry Co.), and as references of polymer, methacrylic acid copolymer LD (article name: Oidragid L30D-55, by Rehmpharma Co., referred to as "L30D-55"), and croscarmellose sodium (article name: Ac-Di-Sol, by Asahi Kasei Co.). In addition, granules of Reference Example 2 were prepared in the same manner except for no stabilizing agent was used.

Test Example 2

The granules of Examples 3 to 5, the granules of Comparative Examples 1 to 4, and the granules of Reference Example 2, which containing mannitol as an excipient and PVP-K30 as a binder, were stored for two or four weeks under 40°C and 75% RH with open conditions and 60°C with closed conditions respectively, then the impurity contents were measured. As a result, it was confirmed that the impurity content of Reference Example 2, which being formulated with no stabilizing agent, increases with time under all of the storage conditions, whereas the impurity contents of Examples, which being formulated with iron oxide or carrageenan, are suppressed to increase with time (see FIGs. 1 and 2). On the other hand, the impurity contents of granules, which being formulated with the other stabilizing agents, represented a higher level than that of Reference Example 2 under a certain storage condition (Table 3) at two weeks of the storage period, thus such test granules were not tested under four weeks of storage period. These results demonstrate that the iron oxide or the carrageenan can provide an effect to suppress the impurity content of amlodipine besylate, which being likely to increase under the effect of temperatures and humidities, in the pharmaceutical composition formulated with an excipient and a binder.

Table 3

| | Stabilizing Agent | 40°C 75%RH Open | | 60°C closed | |
|---|---|---|---|---|---|
| | Storage Period | 2 | 4 | 2 | 4 |
| Ex. 3 | Yellow Ferric Oxide | 0.13% | 0.14% | 0.16% | 0.19% |
| Ex. 4 | Red Ferric Oxide | 0.12% | 0.07% | 0.34% | 0.40% |
| Ex. 5 | Carrageenan | 0.13% | 0.11 % | 0.26% | 0.27% |
| Com. Ex. 1 | Asc | 1.32% | - | 1.34% | - |
| Com. Ex. 2 | Mg hydroxide | 0.94% | - | 1.34% | - |
| Com. Ex. 3 | L30D-55 | 0.50% | 0.88% | 4.94% | - |
| Com. Ex. 4 | Ac-Di-Sol | 0.80% | - | 0.85% | - |
| Ref. Ex. 2 | non-compounded | 0.50% | 0.67% | 0.23% | 0.53% |
| -: not measured | | | | | |

Examples 6 to 12 and Reference Examples 3

Mixtures of amlodipine besylate, mannitol, and yellow ferric oxide or carrageenan were prepared in accordance with the formulation of Table 4 by use of a mechanomill (by Okada Seiko Co.) and then the mixtures were kneaded with an ethanol solution with PVP-K30. Each of the resulting wet powders was molded into tablets by use of an EMP rapidly-disintegrating tablet system (EMT18 and ETD18, disclosed in JP-B No. 3179658) and dried thereby to prepare rapidly disintegrating tablets each containing 3.47 mg of amlodipine besylate and having a unit mass of 170 mg. The tablets were produced in a condition of diameter 8 mm, thickness 3.2 mm, pressing pressure 15 kgf at the press-molding, 70°C of first drying temperature, and 50 °C of second drying temperature. Similarly, rapidly disintegrating tablets were prepared without formulating yellow ferric oxide or carrageenan (Reference Example 3).

Table 4

| | Amlodipine Besylate | Mannitol | Stabilizing Agent |
|---|---|---|---|
| Ex.6 | 10.2 g | 484.3 g | Yellow Ferric Oxide 0.5 g |
| Ex.7 | 10.2 g | 483.8 g | Yellow Ferric Oxide 1.0 g |
| Ex.8 | 10.2g | 479.7 g | Yellow Ferric Oxide 5.1 g |
| Ex. 9 | 10.2 g | 483.8 g | Carrageenan 1.0 g |
| Ex. 10 | 10.2 g | 481.8 g | Carrageenan 3.0 g |
| Ex. 11 | 10.2 g | 480.8 g | Carrageenan 4.0 g |
| Ex. 12 | 10.2 g | 479.7 g | Carrageenan 5.1 g |
| Ref. Ex. 3 | 20.4 g | 969.6 g | - |

Test Example 3

The rapidly disintegrating tablets of Examples 6 to 8 and the rapidly disintegrating tablets of Reference Example 3 were used to investigate with respect to ratio of yellow ferric oxide to amlodipine besylate. The tablets were stored for one month under 60°C with open conditions as the stability test, then the impurity contents were measured. The storage stability test was also conducted under light irradiation. The results are shown in FIG. 3, which represents a relation between ratios of yellow ferric oxide to amlodipine besylate and the impurity content.

Test Example 4

The rapidly disintegrating tablets of Examples 9 to 12 and the tablets of Reference Example 3 were used to investigate with respect to ratio of carrageenan to amlodipine besylate. The tablets were stored for one month under 60°C with open conditions as the stability test, then impurity content was measured. The results are shown in FIG. 4, which represents a relation between ratios of carrageenan to amlodipine besylate and the impurity content.

Example 13

A mixture of 10.2 g of amlodipine besylate, 0.5 g of yellow ferric oxide, 0.5 g of red ferric oxide, and 483.8 g of mannitol was mixed well, and then the resulting mixture was kneaded by an ethanol solution with PVP-K30. Each of the kneaded wet powders was molded into tablets by use of an EMP rapidly-disintegrating tablet system (EMT18 and ETD18, disclosed in JP-B No. 3179658) and dried thereby to prepare rapidly disintegrating tablets each containing 3.47 mg of amlodipine besylate and having a unit mass of 170 mg. The tablets were produced in a condition of diameter 8 mm, thickness 3.2 mm, pressing pressure 15 kgf at the press-molding, 70 °C of first drying temperature, and 50°C of second drying temperature.

Test Example 5

The rapidly disintegrating tablets of Example 13 were used to investigate the stabilization effect with respect to the mixture of yellow ferric oxide and red ferric oxide to amlodipine besylate. , The tablets were stored for one month under 40°C and RH 75% with closed conditions, 60°C with open conditions and light irradiation respectively, then the impurity contents were measured. The results are shown in comparison with Reference Example 3 and Example 7 as shown in Table 5. As a result, it was clearly confirmed that the impurity content of Examples, formulated with iron oxide, was suppressed under influence of higher temperatures, higher humidities, or light irradiation compared to Reference Example 3 formulated with no iron oxide. It was also confirmed that the mixture of yellow ferric oxide and red ferric oxide may exhibit an equivalent effect with yellow ferric oxide itself as a stabilizing agent.

Table 5

| | Compounding Ratio to Amlodipine Besylate | 40°C 75%RH Closed | Light Irradiation | 60°C Open |
|---|---|---|---|---|
| Ex. 13 | Yellow Ferric Oxide (0.05) + Red Ferric Oxide (0.05) | 0.26% | 0.84% | 1.59% |
| Ex. 7 | Yellow Ferric Oxide (0.10) | - | 0.76% | 1.63% |

(continued)

| | Compounding Ratio to Amlodipine Besylate | 40°C 75%RH Closed | Light Irradiation | 60°C Open |
|---|---|---|---|---|
| Ref. Ex. 3 | non-compounded | 0.39% | 1.88% | 2.26% |

Examples 14 to 16 and Reference Example 4

0.35 g of amlodipine besylate, 0.0355 g of yellow ferric oxide, and 17.61 g of a mixture of crystalline cellulose (article name: Avisel PH101, by Asahi Kasei Co.) and mannitol (Avisel/mannitol = 4/6) were mixed well to prepare a mixture, and then the mixture was compressed into tablets through direct punching by use of Shimadzu Autograph AGS-1000D at a tabletting pressure of 300 kgf to prepare tablets of diameter 8 mm and a unit mass of 180 mg of Example 14. In a similar manner with described above, as Example 15, tablets of diameter 8 mm and a unit mass of 180 mg were prepare from 0.35 g of amlodipine besylate, 0.105 g of yellow ferric oxide, and 17.54 g of the mixture described above (Avisel/mannitol = 4/6); and in a similar manner with described above, as Example 16, tablets of diameter 8 mm and a unit mass of 180 mg were prepare from 0.35 g of amlodipine besylate, 0.175 g of yellow ferric oxide, and 17.52 g of the mixture described above (Avisel/mannitol = 4/6). In addition, as Reference Example 4, tablets of diameter 8 mm and a unit mass of 180 mg were prepare from 0.35 g of amlodipine besylate and 17.62 g of the mixture described above (Avisel/mannitol = 4/6) without formulating yellow ferric oxide.

Test Example 6

The tablets of Examples 14 to 16 and the tablets of Reference Example 4 were used to investigate in terms of storage stability under two conditions of under 60°C with open conditions and light irradiation for one month, then impurity contents were measured. Consequently, the impurity contents of Reference Example 4 were 0.08% under the open atmosphere at 60°C and 0.45% under the light irradiation. The impurity contents of Example 14 to 16 were expressed as a ratio based on the impurity content of Reference Example 4 to evaluate comparatively between them as shown in FIG. 5. As a result, it was confirmed that the higher is the compounding ratio, within a range of 0.1 to 0.5, of yellow ferric oxide to amlodipine besylate the lower is the impurity content under every conditions. Therefore, it is demonstrated that the yellow ferric oxide may suppress the decomposition of the dihydropyridine compound and effect to stabilize also in dosage form of tablets. In addition, it was confirmed that the stability of the active ingredient can be improved by direct dry-compression as compared to it the pharmaceutical compositions by produced with an organic solvent.

Examples 17 to 19

0.058 g of amlodipine besylate, 0.029 g of red ferric oxide, and 2.91 g of a mixture of crystalline cellulose (article name: Avisel PH102, referred to as "Avisel", by Asahi Kasei Co.) and mannitol (Avisel/mannitol = 4/6) were mixed well to prepare a mixture, and then the mixture was dry-compressed into tablets through direct punching by use of Shimadzu Autograph AGS-1000D at a tabletting pressure of 300 kgf to prepare tablets of diameter 8 mm and a unit mass of 180 mg of Example 17. In a similar manner with described above, as Example 18, tablets of diameter 8 mm and a unit mass of 180 mg were prepare from 0.058 g of amlodipine besylate, 0.058 g of red ferric oxide, and 2.88 g of the mixture described above (Avisel/mannitol = 4/6); and also in a similar manner with described above, as Example 19, tablets of diameter 8 mm and a unit mass of 180 mg were prepare from 0.058 g of amlodipine besylate, 0.292 g of yellow ferric oxide, and 2.65 g of the mixture described above (Avisel/mannitol = 4/6).

Test Example 7

The tablets of Examples 17 to 19 and the tablets of Reference Example 4 were used to investigate in terms of storage stability under two conditions, or under 60°C with open conditions and light irradiation for one month. Consequently, the impurity content of Reference Example 4 was 0.08% after the open atmosphere at 60°C and 0.45% after the light irradiation. The impurity contents of Example 17 to 19 were expressed as a ratio based on the impurity content of Reference Example 4 to evaluate comparatively between them as shown in FIG. 6. As a result, it was confirmed that the impurity content decreased with the increase in the compounding ratio within a range of the compounding ration from 0.5 to 5, of red ferric oxide to amlodipine besylate the lower is the impurity content under light irradiation. In addition, it was confirmed that the composition of the red ferric oxide may suppress the impurity content under higher humidity.

It became clear that the red ferric oxide can prevent decomposition of the dihydropyridine compound, and have the stability effect in dosage form of tablets also.

Examples 20 to 22 and Reference Example 5

0.04 g of benedipine hydrochloride, 0.004 g of yellow ferric oxide, and 1.756 g of a mixture of crystalline cellulose (article name: Avisel PH102, by Asahi Kasei Co.) and mannitol (Avisel/mannitol = 4/6) were mixed well to prepare a mixture, and then the mixture was dry-compressed into tablets through direct punching by use of Shimadzu Autograph AGS-1000D at a tabletting pressure of 300 kgf to prepare tablets of diameter 8 mm and a unit mass of 180 mg of Example 20. In a similar manner with described above, as Example 21, tablets of diameter 8 mm and a unit mass of 180 mg were prepared from 0.04 g of benedipine hydrochloride, 0.012 g of yellow ferric oxide, and 1.748 g of the mixture described above (Avisel/mannitol = 4/6); and in a similar manner with described above, as Example 22, tablets of diameter 8 mm and a unit mass of 180 mg were prepared from 0.02 g of yellow ferric oxide and 1.74 g of the mixture described above (Avisel/mannitol = 4/6). In addition, as Reference Example 5, tablets of diameter 8 mm and a unit mass of 180 mg were prepared from 0.04 g of benedipine hydrochloride and 1.76 g of the mixture described above (Avisel/mannitol = 4/6) without yellow ferric oxide. Test Example 8

The tablets of Examples 20 to 22 and Reference Example 5 were utilized to investigate the storage stability under an open atmosphere at 60°C for one month and measured the impurity content in accordance with the method shown below.

Measurement of Impurity Content (ii)

The impurity content of the pharmaceutical composition containing benedipine hydrochloride was analyzed as follows. That is, one tablet was put into a measuring flask of 50 mL, to which 10 mL of water and then methanol were added into a total volume of 50 mL, and the mixture was ultrasonically treated well to prepare a sample solution. The sample solution was centrifuged and/or filtered as required (n = 2). HPLC conditions were as follows, i.e. detector: UV absorption spectrophotometer (main measuring wavelength: 237 nm), column temperature: 40°C, column: Inertsil ODS-3 (4.6 mm×15 cm, 5 μm), flow rate: 1.5 mL/min, injection amount: 20 μL, HPLC mobile phase: mixture liquid of 0.05 M phosphate buffer (pH 3.0)/methanol/tetrahydrofuran (65:27:8), analysis period: 30 min.

The test result of Reference Example 5 showed an impurity content of 0.64% for one month under an open atmosphere at 60°C. On the other hand, it was confirmed that the higher is the ratio, within a range of 0.5 to 5, of yellow ferric oxide to benedipine hydrochloride the lower is the impurity content (FIG. 7). Consequently, it is demonstrated that the yellow ferric oxide may suppress the decomposition and effect the stabilization of the dihydropyridine compound.

Industrial Applicability

In accordance with the present invention, a pharmaceutical composition containing a dihydropyridine compound is provided in which the dihydropyridine compound can maintain its stability to lights, moistures, or temperatures even in such dosage forms as having higher surface areas like granules and fine granules or as rapidly disintegrating tablets to which coating being typically unfeasible, even without employing an especial coating agent, a capsule material, or a package by way of compounding the dihydropyridine compound with at least one selected from iron oxide and carrageenan. Therefore, the present invention provides a pharmaceutical composition that can maintain higher quality with substantially no possibility to decrease the content of the amlodipine besylate induced by such reasons as storage conditions of pharmaceutical products till administering to patients or operating conditions to dispense using automatic dose unit packaging machines, or substantially no fear of breakage or pulverization for granules or tablets while their delivery or preparation. In addition, the production method according to the present invention can simplify the production processes and thus the productivity can be enhanced, since there needs no light-shielding coating on the pharmaceutical composition in order to stabilize the amlodipine besylate by way of compounding the amlodipine besylate with at least one selected from yellow ferric oxide, red ferric oxide carrageenan.

Moreover, the stabilizing method and the stabilizing agent according to the present invention can enhance a freedom degree in designing formulation of the pharmaceutical composition, since the degradation amlodipine besylate can be suppressed under various conditions of lights, temperatures, and additives by way of compounding amlodipine besylate at least one selected from yellow ferric oxide, red ferric oxide and carrageenan. Accordingly, the present invention can provide a drug containing amlodipine besylate that can lead to oral rapidly-disintegrating tablets allowing to easily administer to patients with poor swallowing ability or that can lead to granules or scored tablets allowing to easily adjust the dosage levels finely depending on symptom or age of patients.

As such, the pharmaceutical composition according to the present invention can be usable to treat or to remedy hypertension, renoparenchymal hypertension, renovascular hypertension, angina pectoris, and variant angina pectoris of old persons.

## Claims

1. A pharmaceutical composition comprising amlodipine besylate and at least one selected from yellow ferric oxide, red ferric oxide and carrageenan.

2. The pharmaceutical composition according to claim 1, wherein amlodipine besylate and at least one selected from yellow ferric oxide, red ferric oxide and carrageenan are mixed.

3. The pharmaceutical composition according to any one of claims 1 or 2, wherein the content of at least one of yellow ferric oxide and red ferric oxide is 0.05 part by mass to 8 parts by mass based on 1 part by mass of amlodipine besylate.

4. The pharmaceutical composition according to any one of claims 1 to 3 wherein at least one of yellow ferric oxide and red ferric oxide is yellow ferric oxide.

5. The pharmaceutical composition according claim 4, wherein the content of the yellow ferric oxide is 0.05 part by mass to 1 part by mass based on 1 part by mass of amlodipine besylate.

6. The pharmaceutical composition according to any one of claims 1 to 5, wherein the content of the carrageen is 0.05 part by mass to 1.2 parts by mass based on 1 part by mass of amlodipine besylate.

7. The pharmaceutical composition according to any one of claims 1 to 6, wherein the pharmaceutical composition is one of fine granules, tablets, capsules, and dry syrups.

8. The pharmaceutical composition, according to claim 7, wherein the pharmaceutical composition is in the form of tablets, obtainable by a method comprising:

    (i) mixing amlodipine besylate and at least one selected from yellow ferric oxide, red ferric oxide and carrageenan to prepare a mixture, and
    (ii) dry-compressing the mixture into tablets.

9. The pharmaceutical composition, according to claim 7 or 8, wherein the tablets are rapidly disintegrating tablets.

10. A method for producing a pharmaceutical composition, comprising:

    (i) mixing amlodipine besylate and at least one selected from yellow ferric oxide, red ferric oxide and carrageenan to prepare a mixture, and
    (ii) dry-compressing the mixture into tablets.

11. A method for stabilizing amlodipine besylate in a pharmaceutical composition, comprising mixing amlodipine besylate and at least one selected from yellow ferric oxide, red ferric oxide and carrageenan.

## Patentansprüche

1. Pharmazeutische Zusammensetzung, umfassend Amlodipinbesylat und mindestens eines ausgewählt aus gelbem Eisen(III)oxid, rotem Eisen(III)oxid und Carrageenan.

2. Pharmazeutische Zusammensetzung gemäß Anspruch 1, worin Amlodipinbesylat und mindestens eines, ausgewählt aus gelbem Eisen(III)oxid, rotem Eisen(III)oxid und Carrageenan, gemischt sind.

3. Pharmazeutische Zusammensetzung gemäß einem der Ansprüche 1 oder 2, worin der Gehalt von mindestens einem aus gelbem Eisen(III)oxid und rotem Eisen(III)oxid 0,05 Gew.-Teile bis 8 Gew.-Teile, bezogen auf 1 Gew.-Teil Amlodipinbesylat, beträgt.

4. Pharmazeutische Zusammensetzung gemäß einem der Ansprüche 1 bis 3, worin mindestens eines aus gelbem Eisen(III)oxid und rotem Eisen(III)oxid gelbes Eisen(III)oxid ist.

5. Pharmazeutische Zusammensetzung gemäß Anspruch 4, worin der Gehalt des gelben Eisen(III)oxids 0,05 Gew.-Teile bis 1 Gew.-Teil, bezogen auf 1 Gew.-Teil Amlodipinbesylat, beträgt.

6. Pharmazeutische Zusammensetzung gemäß einem der Ansprüche 1 bis 5, worin der Gehalt des Carrageens 0,05 Gew.-Teile bis 1,2 Gew.-Teile, bezogen auf 1 Gew.-Teil Amlodipinbesylat, beträgt.

7. Pharmazeutische Zusammensetzung gemäß einem der Ansprüche 1 bis 6, worin die pharmazeutische Zusammensetzung eine aus feinen Granalien, Tabletten, Kapseln und Trockensäften ist.

8. Pharmazeutische Zusammensetzung gemäß Anspruch 7, worin die pharmazeutische Zusammensetzung in Form von Tabletten vorliegt, erhältlich mittels eines Verfahrens, umfassend:

(i) Mischen von Amlodipinbesylat und mindestens einem, ausgewählt aus gelbem Eisen(III)oxid, rotem Eisen (III)oxid und Carrageenan, um eine Mischung herzustellen, und
(ii) Trockenverpressen der Mischung zu Tabletten.

9. Pharmazeutische Zusammensetzung gemäß Anspruch 7 oder 8, worin die Tabletten schnell zerfallende Tabletten sind.

10. Verfahren zur Herstellung einer pharmazeutischen Zusammensetzung, umfassend:

(i) Mischen von Amlodipinbesylat und mindestens einem, ausgewählt aus gelbem Eisen(III)oxid, rotem Eisen (III)oxid und Carrageenan, um eine Mischung herzustellen, und
(ii) Trockenverpressen der Mischung zu Tabletten.

11. Verfahren zur Stabilisierung von Amlodipinbesylat in einer pharmazeutischen Zusammensetzung, umfassend das Mischen von Amlodipinbesylat und mindestens einem, ausgewählt aus gelbem Eisen(III)oxid, rotem Eisen(III)oxid und Carrageenan.

## Revendications

1. Composition pharmaceutique comprenant du bésylate d'amlodipine et au moins un choisi parmi l'oxyde de fer (III) jaune, l'oxyde de fer (III) rouge et le carraghénane.

2. Composition pharmaceutique selon la revendication 1, dans laquelle le bésylate d'amlodipine et au moins un choisi parmi l'oxyde de fer (III) jaune, l'oxyde de fer (III) rouge et le carraghénane sont mélangés.

3. Composition pharmaceutique selon l'une quelconque des revendications 1 ou 2, dans laquelle la teneur de au moins un parmi l'oxyde de fer (III) jaune et l'oxyde de fer (III) rouge est de 0,05 partie en masse à 8 parties en masse sur la base de 1 partie en masse de bésylate d'amlodipine.

4. Composition pharmaceutique selon l'une quelconque des revendications 1 à 3, dans laquelle au moins un parmi l'oxyde de fer (III) jaune et l'oxyde de fer (III) rouge est l'oxyde de fer (III) jaune.

5. Composition pharmaceutique selon la revendication 4, dans laquelle la teneur de l'oxyde de fer (III) jaune est de 0,05 partie en masse à 1 partie en masse sur la base de 1 partie en masse de bésylate d'amlodipine.

6. Composition pharmaceutique selon l'une quelconque des revendications 1 à 5, dans laquelle la teneur du carragahénane est de 0,05 partie en masse à 1,2 parties en masse sur la base de 1 partie en masse de bésylate d'amlodipine.

7. Composition pharmaceutique selon l'une quelconque des revendications 1 à 6, dans laquelle la composition pharmaceutique est une parmi les granulés fins, les comprimés, les capsules, et les sirops secs.

8. Composition pharmaceutique selon la revendication 7, dans laquelle la composition pharmaceutique est sous la forme de comprimés, pouvant être obtenus par un procédé comprenant :

(i) le mélange de bésylate d'amlodipine et d'au moins un choisi parmi l'oxyde de fer (III) jaune, l'oxyde de fer (III) rouge et le carraghénane pour préparer un mélange, et
(ii) la compression à sec du mélange en comprimés.

**9.** Composition pharmaceutique selon la revendication 7 ou 8, dans laquelle les comprimés sont des comprimés se désagrégeant rapidement.

**10.** Procédé pour produire une composition pharmaceutique comprenant :

(i) le mélange de bésylate d'amlodipine et d'au moins un choisi parmi l'oxyde de fer (III) jaune, l'oxyde de fer (III) rouge et le carraghénane pour préparer un mélange, et
(ii) la compression à sec du mélange en comprimés.

**11.** Procédé pour stabiliser le bésylate d'amlodipine dans une composition pharmaceutique, comprenant le mélange de bésylate d'amlodipine et d'au moins un choisi parmi l'oxyde de fer (III) jaune, l'oxyde de fer (III) rouge et le carraghénane.

FIG. 1

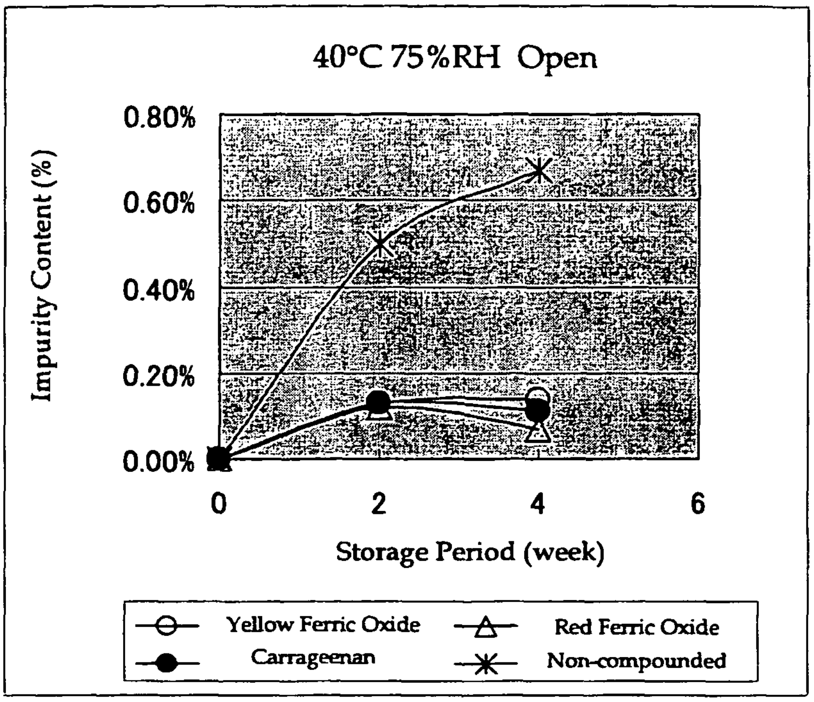
40°C 75%RH Open
Impurity Content (%)
0.80%
0.60%
0.40%
0.20%
0.00%
0
2
4
6
Storage Period (week)
Yellow Ferric Oxide
Red Ferric Oxide
Carrageenan
Non-compounded

FIG. 2

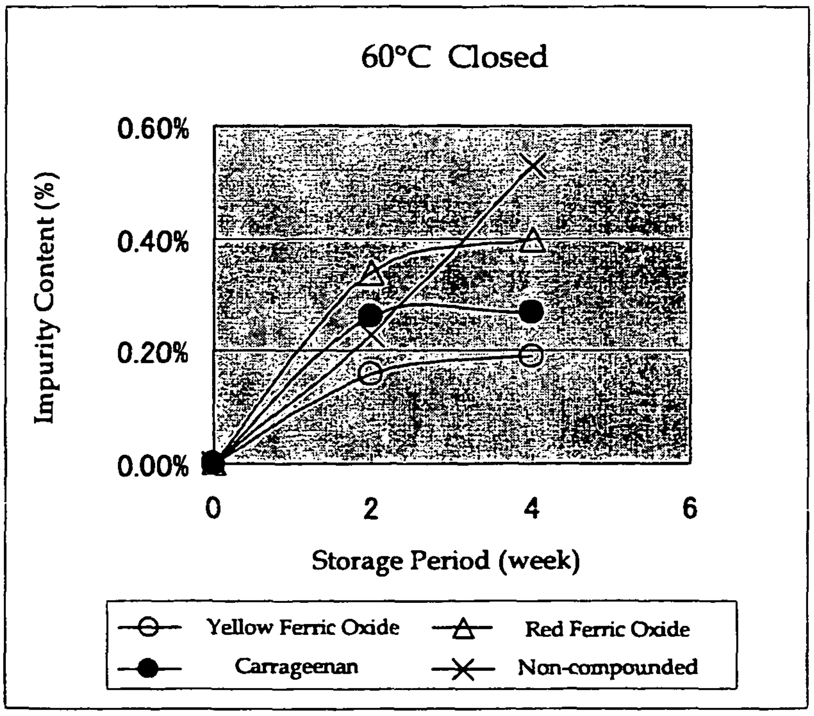
60°C Closed
Impurity Content (%)
0.60%
0.40%
0.20%
0.00%
0
2
4
6
Storage Period (week)
Yellow Ferric Oxide
Red Ferric Oxide
Carrageenan
Non-compounded

FIG. 3

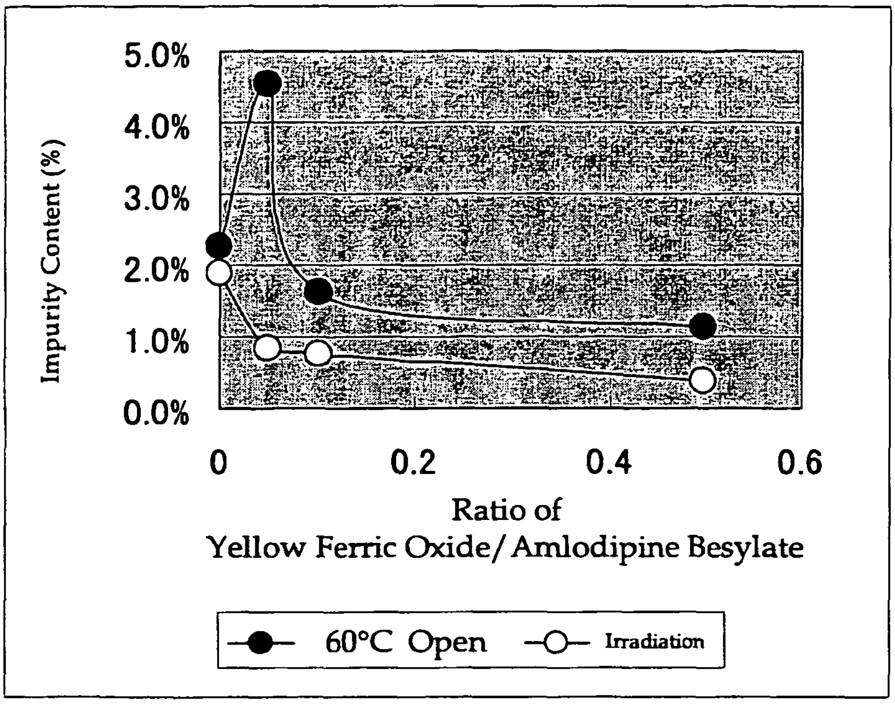
5.0%
4.0%
3.0%
2.0%
1.0%
0.0%
Impurity Content (%)
0
0.2
0.4
0.6
Ratio of
Yellow Ferric Oxide/Amlodipine Besylate
60°C Open
Irradiation

FIG. 4

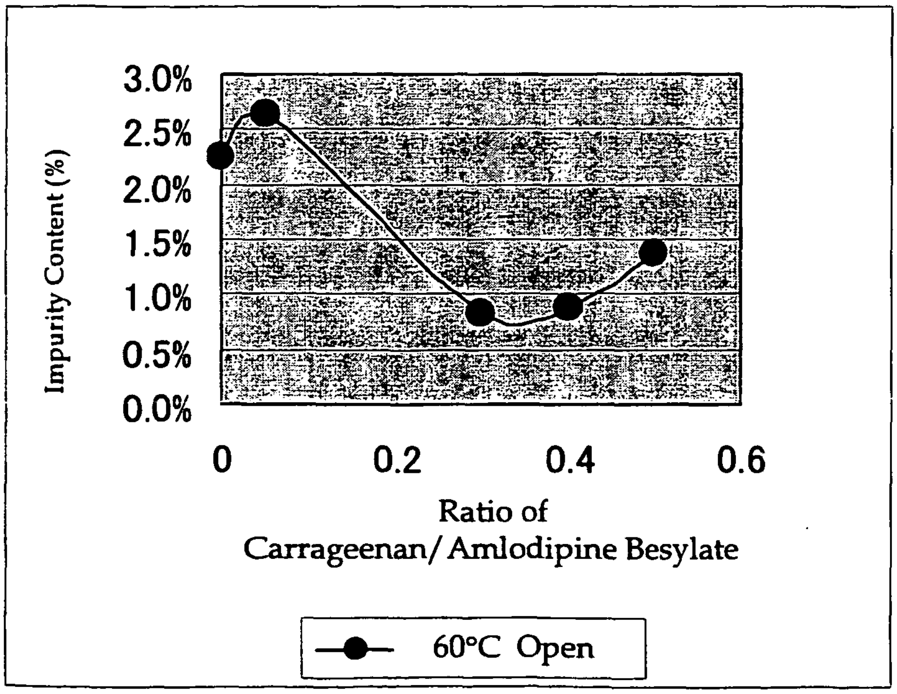
3.0%
2.5%
2.0%
1.5%
1.0%
0.5%
0.0%
Impurity Content (%)
0
0.2
0.4
0.6
Ratio of
Carrageenan/Amlodipine Besylate
60°C Open

FIG. 5

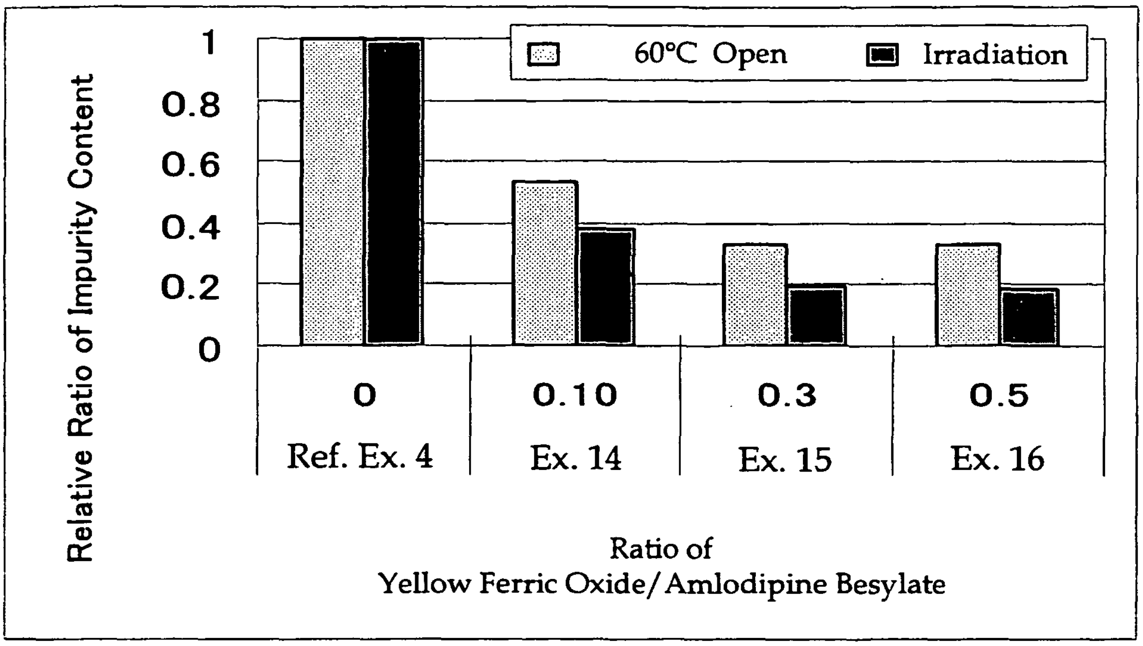
60°C Open
Irradiation
Relative Ratio of Impurity Content
1
0.8
0.6
0.4
0.2
0
0
Ref. Ex. 4
0.10
Ex. 14
0.3
Ex. 15
0.5
Ex. 16
Ratio of
Yellow Ferric Oxide/Amlodipine Besylate

FIG. 6

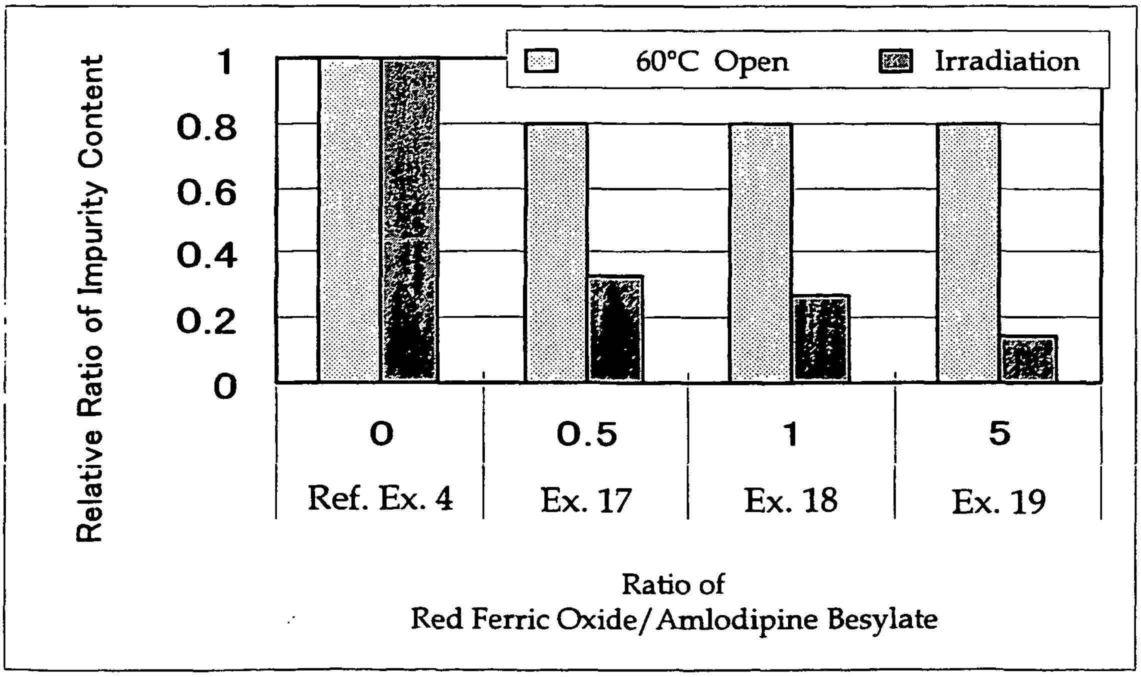
60°C Open
Irradiation
Relative Ratio of Impurity Content
1
0.8
0.6
0.4
0.2
0
0
Ref. Ex. 4
0.5
Ex. 17
1
Ex. 18
5
Ex. 19
Ratio of
Red Ferric Oxide/Amlodipine Besylate

FIG. 7

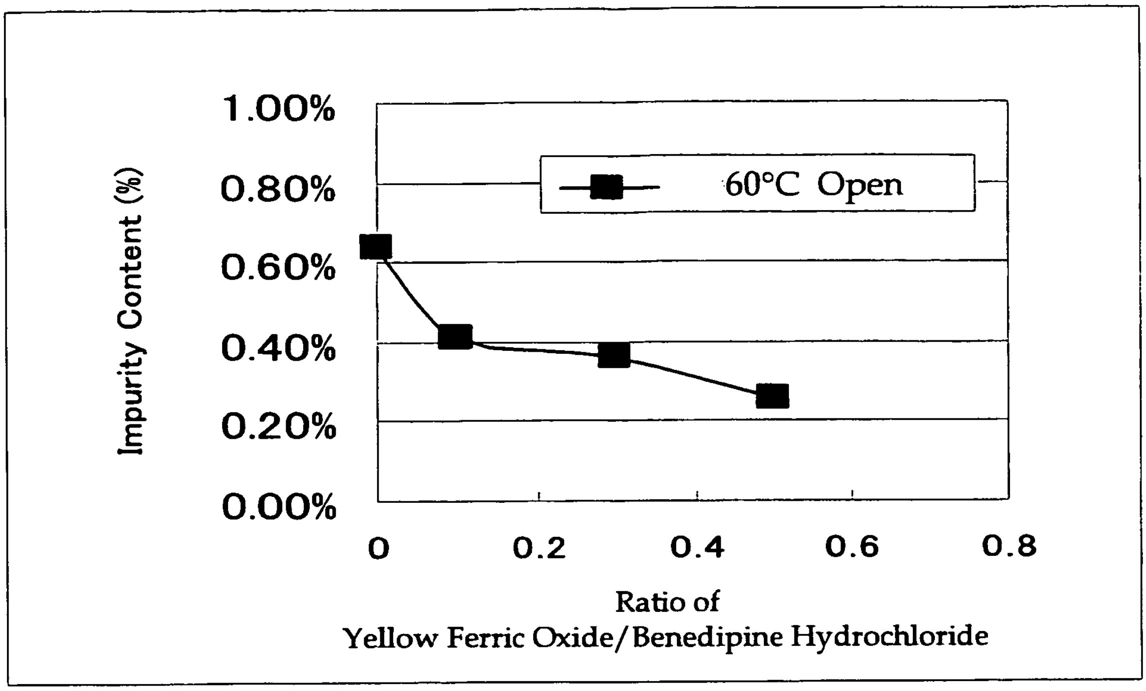
1.00%
0.80%
0.60%
0.40%
0.20%
0.00%
Impurity Content (%)
60°C Open
0
0.2
0.4
0.6
0.8
Ratio of
Yellow Ferric Oxide/Benedipine Hydrochloride

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003104888 A
- WO 03051364 A
- JP 57058609 A
- JP 2000191516 A
- JP 3179658 B


### Non-patent literature cited in the description


- **M. Abdoh et al.** Amlodipine Besylate-Excipients Interaction in Solid Dosage Form. *Pharmaceutical Development and Technology, USA,* 2004, vol. 9 (1), 15-24
- **Hiroshi Yuasa et al.** Tablet Discoloration under Light as well as BHT. *Hospital and Pharmacy,* 1995, vol. 21 (3), 231-242
- **Desai D.S. et al.** Photostabilization of uncoated tablets of sorivudine and nifedipine by incorporation of synthetic iron oxides. *INTERNATIONAL JOURNAL OF PHARMACEUTICS,* 25 February 1994, vol. 103 (1), 69-76
- **Desai D.S. et al.** Photostabilization of the tablets of antiviral drug SQ32756 (BV-araU) and nifedipine by incorporation of synthetic iron oxides. *PHARMACEUTICAL RESEARCH,* October 1991, vol. 8 (10), S-176
- Japanese Pharmacopoeia, Pharmaceutical Additives Standards, Japanese Standards for Pharmaceutical Ingredients. *Food Additives, or reagents,* 1997